# EUROPÄISCHE PATENTSCHRIFT

(11) **EP 1 124 534 B1**
(45) Veröffentlichungstag und Bekanntmachung des Hinweises auf die Patenterteilung: **14.01.2004**
(21) Anmeldenummer: 99971306.8
(22) Anmeldetag: 23.10.1999
(51) Int. Cl.: A61K 9/00

(54) **EXPANDIERBARES GASTRORETENTIVES THERAPIE-SYSTEM MIT VERLÄNGERTER MAGENVERWEILDAUER**
EXPANDABLE GASTRORETENTIVE THERAPEUTICAL SYSTEM WITH PROLONGED STOMACH RETENTION TIME
SYSTEME THERAPEUTIQUE GONFLANT DE RETENTION STOMACALE A TEMPS DE SEJOUR PROLONGE DANS L'ESTOMAC

(30) Priorität: 30.10.1998 DE 19850309
(43) Veröffentlichungstag der Anmeldung: 22.08.2001
(73) Patentinhaber: LTS LOHMANN THERAPIE-SYSTEME GmbH & CO.KG, 56626 Andernach (DE)
(72) Erfinder: KRUMME, Markus, D-56567 Neuwied (DE)
(74) Vertreter: Schmidt, Werner, Dr.
(86) Internationale Anmeldenummer: PCT/EP1999/008043
(87) Internationale Veröffentlichungsnummer: WO 2000/025742

(56) Entgegenhaltungen:
- FR-A- 2 323 440

## Beschreibung

Die vorliegende Erfindung beschreibt eine Vorrichtung zur Verzögerung der Pyloruspassage oral einnehmbarer Arzneiformen mit einer bei Kontakt mit Magensaft expandierbaren Komponente und einer für Flüssigkeiten durchlässigen jedoch gasdichten Polymerhülle. Die Vorrichtung kann einen Wirkstoff enthalten, der überwiegend von der inkorporierten Arzneiform gesteuert in den Magensaft abgegeben wird. Gegenüber herkömmlichen Arzneiformen mit verzögerter Pyloruspassage ist die Wirkstofffreisetzung weniger abhängig von Art und Beschaffenheit der Polymerhülle und wird überwiegend von der inkorporierten Form bestimmt. Die erfindungsgemässe Vorrichtung läßt sich leicht rollen oder falten und kann in Kapseln verfüllt werden.

Aus FR 2 323 440 ist eine Vorrichtung zur Abgabe von Wirkstoffen an eine flüssigkeitshaltige Umgebung durch Osmose bekannt, die eine Wand aus einem für den Wirkstoff undurchlässigen Material enthält. Ein bei Berührung mit Flüssigkeit aktiviertes gaserzeugendes Mittel bewirkt den Austritt des Wirkstoffs durch eine Austrittsöffnung in dieser Wand.

In der US-PS 4,207,890 wird eine Vorrichtung zur kontrollierten Freisetzung von Wirkstoffen beschrieben, die durch ihre Expansion eine lokale Retention im Magen erfährt und dadurch eine verlängerte Verweilzeit im Magen aufweist. Die Vorrichtung weist (a) eine Polymerhülle auf, die vor der Applikation kollabiert vorliegt. Die Polymerhülle selbst besitzt keine Öffnungen und besteht aus einem Material, das praktisch nicht hydratisierbar , jedoch für Körperflüssigkeiten permeabel ist. Die Vorrichtung weist außerdem (b) ein Element auf, welches die Wirkstoffabgabe steuert. Dieses Element kann nach Anspruch 2 die Polymerhülle selbst sein. Als weiteres Element (c) besitzt die Vorrichtung eine bei Kontakt mit Körperflüssigkeiten expandierende Komponente.

Das treibende Prinzip der retentiven Wirkung ist die Osmose, wobei das Element (c) eine osmotisch aktive Substanz darstellt die Wasser durch die Polymerhülle (a) treibt und dadurch das Ausbilden einer Lösung in der Vorrichtung bedingt. Die Vorrichtung wird so im expandierten Zustand eine wässrige Lösung enthalten und aufgrund der Konzentrationsverhältnisse eine Wirkstoffausdiffusion aus der Vorrichtung ermöglichen, jedoch in ihrer scheinbaren Dichte in etwa dem Speisebrei entsprechen. Damit wird die Vorrichtung im Speisebrei schweben oder sinken. Retentives Prinzip ist ausschließlich die Größe der Vorrichtung. Ein möglicher Deflationsmechanismus wird nicht beschrieben, damit ist die Entfernung der Vorrichtung aus dem Magen und die Entsorgung nach Wirkstoffabgabe nicht geklärt.

Eine Modifikation des Retentionsprinzips ist in der EP 0 307 904 A1 und der US-PS 4,996,058 gezeigt. Als expandierbare Komponente sind Stoffe angegeben, die unter Säurezutritt gasförmige Komponenten wie Kohlendioxid oder Stickstoff bilden, die die Polymerhülle (a) aufblähen und so eine auf dem Speisebrei schwimmfähige Vorrichtung bilden. Das Retentionsprinzip ist (s. US-PS 4,996,058 Spalte 3, Zeilen 6 bis 7) Aufschwimmen auf dem Speisebrei und damit wird die Vorrichtung vor der Entleerung des Magens durch den Pylorus bewahrt. Diese Vorrichtungen haben entscheidende Nachteile. Die Steuerung der Wirkstoffabgabe erfolgt in beiden Fällen im wesentlichen durch die Polymerhülle. Gleichzeitig ist die Inflationskinetik der Vorrichtung im wesentlichen durch die Polymerhülle begrenzt, da Wasser wie auch Hydroniumionen durch die Polymerhülle diffundieren müssen, andererseits aber Wirkstoffmoleküle in entgegengesetzter Richtung herausdiffundieren müssen. Die Diffusionskonstante in der Polymerhülle ist ein entscheidender Parameter. Bei Verletzung der relativ dünnen und damit sensiblen Polymerhülle durch mechanische oder chemische Einwirkung ist die Gefahr einer schlagartigen Freigabe des Wirkstoffes im Sinne eines dose-dumping gegeben. Das Maß der produzierten expandierenden Gasmenge hängt unter anderem stark von der Acidität der Umgebung ab, damit aber von der Zusammensetzung des Speisebreies und der Sezemierung der Magensäure. Bei stark saurer Umgebung und damit hoher Gasproduktion darf die mechanische Belastung der Vorrichtungen nicht die Berstgrenze erreichen, andererseits muß bei weniger saurer Umgebung eine ausreichende Gasproduktion erreicht werden. Dieses Dilemma führt zu stark variablen Zuständen der Vorrichtungen infolge von zirkadianen Variationen der Magenacidität.

Der Erfindung liegt daher die Aufgabe zugrunde, ein gastroretentives System zur Verfügung zu stellen, das diese Nachteile der im Stand der Technik bekannten Systeme vermeidet.

Die erfindungsgemässe Lösung dieser Probleme gelingt durch eine gastroretentive Vorrichtung, die folgende Elemente enthält:
(a) eine Polymerhülle aus mikroporöser Membran oder partiell mikroporöser Membran oder einer Kombination aus beiden mit einem anderen nicht-porösen folienartigen Polymer. Die Herstellung der Poren kann erfindungsgemäß durch beliebige Verfahren erfolgen, z. B. durch Recken von Folien, durch Verwendung von Mehrphasensystemen und Evaporation eines Teiles des Systems, durch gezielte Polymerisation z. B. in Form von lonomeren, durch mechanische Verfahren wie Nadeln, durch thermische Verfahren wie Lasern, durch Bestrahlung mit ionisierenden Strahlen und anschließendes Ätzen etc. Als Materialien für die mikroporöse Membran können erfindungsgemäß verwendet werden: Polyurethane, Polypropylen. Polyvinylalkohol, Polyvinylacetat, Polyacrylsäure und Derivate, Polymethylmethacrylsäure und Derivate, Polykarbonate, Polyvinylidendifluorid, Polytetrafluorethylen und beliebige weitere Polymere, die mit Poren geeigneter Größe versehen werden können. Die Größe dieser Poren liegt erfindungsgemäß zwischen 0,1 und 20 µm, bevorzugt zwischen 0,3 und 10 µm und besonders bevorzugt zwischen 0,5 und 1 µm. Um die Poren ggf. besser benetzbar zu machen, können die Membranen erfindungsgemäß mit Netzmitteln oder sonstigen hydrophilen Komponenten getränkt sein.
(b) eine expandierbaren Komponente, die bei Kontakt mit Magensaft, insbesondere unter Säureeinwirkung ein Gas wie z. B. Kohlendioxid oder Stickstoff produziert. Als Beispiele kommen hierfür erfindungsgemäß z. B. Carbonate und Hydrogencarbonate der Alkali- und Erdalkalimetalle, der Ammoniumkationen oder Natriumazid oder Mischungen davon zum Einsatz. Diese expandierbaren Komponenten können zur Modifikation der Gasproduktionskinetik ggf. modifiziert werden, z. B. durch Beschichten mit oder Einbetten in lipophilen Komponenten wie Wachsen oder Fetten oder geeigneten Überzügen wie Polymethacrylaten oder Polymethylmethacrylaten und Derivaten oder ähnlichen dem Fachmann bekannten Substanzen. Die expandierbare Komponente kann erfindungsgemäß auch schaumbildend, z. B. durch Einarbeiten in Polyvinylalkohol, oder in Form einer halbfesten, salbenartigen Zubereitung vorliegen, um die Deflationskinetik zu steuern.
(c) eine den Wirkstoff enthaltende Komponente wie z. B. multipartikuläre Zubereitungen, Tabletten. Kapseln oder halbfeste salbenartige Zubereitungen, oder Schäume. Als Wirkstoff kommen grundsätzlich alle Stoffe mit einer physiologischen Wirkung in Frage, insbesondere Arzneistoffe für die Human- oder Veterinärmedizin, vorzugsweise solche, die durch die Magenschleimhaut resorbiert werden oder an der Oberfläche der Magenschleimhaut wirken. Solche pharmazeutischen Wirkstoffe sind dem Fachmann bekannt. Die Komponenten (b) und (c) können auch in einer gemeinsamen Zubereitung vorliegen.

Die Vorrichtung kann in ein Behältnis aus physiologisch annehmbaren Material, beispielsweise in eine Hartgelatinekapsel, gefüllt werden um die Applikation und die Handhabung zu erleichtern.

Die aufgeblähte Form der Vorrichtung ist vorzugsweise die eines aufgeblasenen Dreieckes um eine planare, maximal sperrige Struktur zu haben. Durch die Planarität sind im auf dem Speisebrei schwimmenden Zustand zwei Vorzugslagen stabil, die beide eine Passage des Speisebreies an der gastroretentiven Vorrichtung vorbei erlauben, so daß der Speisebrei die aufgeblähte Form nicht durch den Pylorus pressen kann. Die Form eines Dreieckes stellt unter den planaren Formen einen guten Kompromiß aus Sperrigkeit und Steifigkeit der Struktur im aufgeblähten Zustand dar und wird daher für die retentive Vorrichtung vorgeschlagen. Im Magen verkeilt sich die sperrige Struktur zwischen den Magenwänden und läßt den Speisebrei durch den Pylorus entweichen, die Verkeilung im Lumen muß nur dem Strömungswiderstand im Speisebrei standhalten. Je kugelförmiger die Vorrichtung ist, desto eher ist eine kolbenartige Abdichtung des Magenausgangs denkbar, mit der Folge, daß der Entleerungsdruck die Vorrichtung durch den Pylorus presst. Erfindungsgemäß können aber auch vier- und mehreckige Strukturen sowie solche mit abgerundeten Ecken verwendet werden.

Durch die Verwendung der mikroporösen oder porösen Polymerhülle gelingt erfindungsgemäß die Lösung mehrerer Probleme. Durch die Oberflächenspannung flüssiger, insbesondere wässriger Systeme ist zur Entleerung einer flüssigkeitsgefüllten Pore ein bestimmter Druck notwendig, der im wesentlichen von Porendurchmesser, Oberflächenspannung und Grenzflächenspannung und dem Kontaktwinkel abhängt. Durch geeignete Wahl des Porendurchmessers läßt sich für ein gegebenes System ein definierter Druck einstellen, unterhalb dessen die Poren nicht entleert werden können. Damit ist die Membran gasdicht. Flüssigkeiten können jedoch leicht durch die Membran fließen, der Flux ist durch die Porenanzaht und die Abmessungen der Poren sowie die Viskosität des Mediums gegeben. Durch Verwendung mikroporöser Membranen definierter Porengröße kann damit erfindungsgemäß eine für Flüssigkeiten hochdurchlässige, für Gase unterhalb eines definierten Druckes jedoch undurchlässige Vorrichtung realisiert werden. Dadurch ist der Innendruck der Vorrichtung auf einen vorgegebenen Wert begrenzt. Bei Überschreiten des Grenzdruckes wird die Membran durchlässig und verhindert als nichtzerstörende Sicherung eine mechanische Belastung der Vorrichtung über den zulässigen Berstdruck hinaus. Damit ist es mit der erfindungsgemäßen Vorrichtung möglich, einen nahezu konstanten Innendruck über die Gebrauchsdauer der Vorrichtung zu erreichen. Die Gebrauchsdauer der, Vorrichtung ist durch die Art und Menge der expandierbaren Komponente steuerbar. Durch Kapillarität werden auf der Innenseite der Membran liegende Partikel benetzt und setzen den Wirkstoff durch die mikroporöse Membran mit nur geringer Verzögerung frei.

Die erfindungsgemäßen Systeme können in Human- und Veterinärmedizin eingesetzt werden.

### Abbildungen

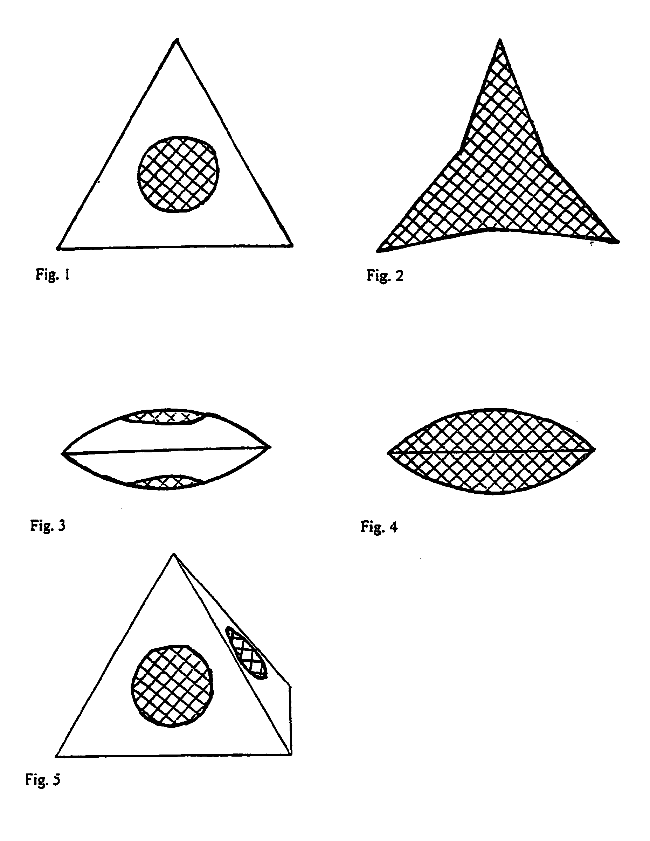

Fig. 1 zeigt den Aufbau eines erfindungsgemäßen Systems in der Aufsicht.
Fig. 2 zeigt den Aufbau eines weiteren erfindungsgemäßen Systems in der Aufsicht.
Fig. 3 zeigt die Seitenansicht des Systems von Fig. 1
Fig. 4 zeigt die Seitenansicht des Systems von Fig. 2
Fig. 5 zeigt perspektivisch ein gastroretentives System in Tetraederform.

In den Abbildungen entsprechen schraffierte Bereiche der mikroporösen Membran, nicht-schraffierte Bereiche entsprechen einer nicht-porösen Polymerfolie. Die Komponenten (b) und (c) sind, weil sie in der Vorrichtung eingeschlossen sind, nicht sichtbar.

## Patentansprüche

1. Gastroretentive Vorrichtung enthaltend eine bei Kontakt mit Magensaft durch Gasbildung expandierbare Komponente und eine einen Wirkstoff enthaltende Komponente, **dadurch gekennzeichnet, daß** diese Komponenten ganz von einer Polymerhülle aus einer mikroporösen oder partiell mikroporösen Membran umgeben sind.

2. Gastroretentive Vorrichtung nach Anspruch 1, **dadurch gekennzeichnet, daß** die Form der Vorrichtung angenähert planar dreieckig oder planar dreizackig sternförmig oder tetraedrisch ist.

3. Gastroretentive Vorrichtung nach Anspruch 1 oder 2, **dadurch gekennzeichnet, daß** die Zubereitung mindestens eines Teiles der expandierbaren Komponente oder mindestens eines Teiles der einen Wirkstoff enthaltenden Komponente oder beider halbfest salbenartig ist.

4. Gastroretentive Vorrichtung nach Anspruch 1 oder 2, **dadurch gekennzeichnet, daß** die Zubereitung mindestens eines Teils der expandierbaren Komponente und / oder mindestens eines Teils der einen Wirkstoff enthaltenden Komponente in einer multipartikulären Zubereitung vorliegt.

5. Gastrotetentive Vorrichtung nach Anspruch 1 oder 2, **dadurch gekennzeichnet, daß** die Zubereitung mindestens eines Teiles der expandierbaren Komponente oder mindestens eines Teiles der einen Wirkstoff enthaltenden Komponente oder beider in einer schaumartigen oder schäumenden Zubereitung vorliegt.

6. Gastrotetentive Vorrichtung nach Anspruch 1 oder 2, **dadurch gekennzeichnet, daß** die Zubereitung mindestens eines Teils der expandierbaren Komponente und / oder mindestens eines Teils der einen Wirkstoff enthaltenden Komponente in einer langsam löslichen kristallographischen Form vorliegt.

7. Gastroretentive Vorrichtung nach einem der vorangegangen Ansprüche, **dadurch gekennzeichnet daß** sie in einer im Magensaft schnell zerfallenden Umhüllung, vorzugsweise einer Hartgelatinekapsel enthalten ist.

8. Gastrotetentive Vorrichtung nach einem der vorangegangenen Ansprüche, **dadurch gekennzeichnet, daß** sie gefaltet und mittels einer magensaftlöstichen Polymerhülle in der gefalteten Form fixiert wird.

## Claims

1. A gastroretentive device comprising a component which is expandable by gas formation on contact with gastric juice, and a component containing an active compound, wherein these components are completely surrounded by a polymer shell comprising a microporous or partially microporous membrane.

2. The gastroretentive device as claimed in claim 1, wherein the form of the device is approximately planar triangular or planar tridentate stellate or tetrahedral.

3. The gastroretentive device as claimed in claim 1 or 2, wherein at least one part of the expandable component or at least one part of the component containing an active compound or both is present in semisolid ointment-like form.

4. The gastroretentive device as claimed in claim 1 or 2, wherein at least one part of the expandable component and/or at least one part of the component containg an active compound is present in a multiparticulate preparation.

5. The gastroretentive device as claimed in claim 1 or 2, wherein at least one part of the expandable component or at least one part of the component containing an active compound or both is present in a foam-like or foaming preparation.

6. The gastroretentive device as claimed in claim 1 or 2, wherein at least one part of the expandable component and/or at least one part of the component containing an active compound is present in a slowly soluble crystallographic form.

7. The gastroretentive device as claimed in one of the preceding claims, which is contained in a coating rapidly disintegrating in gastric juice, preferably a hard gelatin capsule.

8. The gastroretentive device according to one of the preceding claims, which is folded and fixed in the folded form by means of a gastric juice-soluble polymer coat.

## Revendications

1. Dispositif à rétention gastrique, contenant un composant expansible après contact avec le suc gastrique, sous l'effet de la formation d'un gaz, et un composant contenant un principe actif, **caractérisé en ce que** ces composants sont entièrement entourés d'une gaine polymère constituée d'une membrane microporeuse ou partiellement microporeuse.

2. Dispositif à rétention gastrique selon la revendication 1, **caractérisé en ce que** la forme du dispositif est approximativement triangulaire plane ou tridentée plane, en étoile ou tétraédrique.

3. Dispositif à rétention gastrique selon la revendication 1 ou 2, **caractérisé en ce que** la préparation d'au moins une partie de la composition expansible ou d'au moins une partie du composant contenant un principe actif, ou des deux, est semi-solide et du type pommade.

4. Dispositif à rétention gastrique selon la revendication 1 ou 2, **caractérisé en ce que** la préparation d'au moins une partie du composant expansible et/ou d'une partie du composant contenant un principe actif se présente sous forme d'une préparation multiparticulaire.

5. Dispositif à rétention gastrique selon la revendication 1 ou 2, **caractérisé en ce que** la préparation d'au moins une partie du composant expansible ou d'au moins une partie du composant contenant un principe actif, ou des deux, est présente dans une préparation de type mousse, ou moussante.

6. Dispositif à rétention gastrique selon la revendication 1 ou 2, **caractérisé en ce que** la préparation d'au moins une partie du composant expansible et/ou d'au moins une partie du composant contenant un principe actif se présente sous une forme cristallographique lentement soluble.

7. Dispositif à rétention gastrique selon l'une des revendications précédentes, **caractérisé en ce qu'**il est contenu dans un gainage, se décomposant rapidement dans le suc gastrique, par exemple une gélule en gélatine dure.

8. Dispositif à rétention gastrique selon l'une des revendications précédentes, **caractérisé en ce qu'**il est replié et est fixé sous forme repliée à l'aide d'une gaine polymère soluble dans le suc gastrique.
